# EUROPEAN PATENT APPLICATION

(11) **EP 4 245 236 A1**
(43) Date of publication of application: **20.09.2023**
(21) Application number: 21905246.1
(22) Date of filing: 13.10.2021
(51) Int. Cl.: A61B 18/12, A61M 5/142, A61M 5/36, A61M 31/00, A61F 7/00, A61F 7/12

(54) **SYRINGE-PUMP BUBBLE EMPTYING CONTROL METHOD AND DEVICE, SYRINGE PUMP AND STORAGE MEDIUM**

(30) Priority: 16.12.2020 CN 202011489488
(71) Applicant: Hangzhou Broncus Medical Co., Ltd., Hangzhou, Zhejiang 310051 (CN)
(72) Inventor: XU, Hong, Hangzhou, Zhejiang 310051 (CN); LI, Yuhang, Hangzhou, Zhejiang 310051 (CN); SUI, Hailong, Hangzhou, Zhejiang 310051 (CN); JIANG, Lifang, Hangzhou, Zhejiang 310051 (CN); HUANG, Siyuan, Hangzhou, Zhejiang 310051 (CN)
(74) Representative: Krauns, Christian
(86) International application number: PCT/CN2021/123422
(87) International publication number: WO 2022/127317

(57) **Abstract**

A syringe-pump bubble discharging control method and device, a syringe pump and a computer-readable storage medium are disclosed. The method includes detecting whether there is a bubble in a syringe barrel of a syringe pump by means of a bubble detection device; determining the position of the bubble if there is a bubble is in the syringe barrel; and according to the position of the bubble, controlling the syringe pump to perform a discharging operation so as to discharge the bubble from the syringe barrel. The syringe-pump bubble discharging control method and device, the syringe pump and the computer-readable storage medium can realize the automatic control of discharging bubbles in the syringe pump, and not only improves the timeliness of discharging bubbles but also avoids the wasting of a liquid in the syringe barrel and improves the efficiency of the bubble discharging operation.

## Description

### TECHNICAL FIELD

The embodiments of the present disclosure relate to the field of communication technologies, and in particular, to a syringe-pump bubble discharging control method and device, syringe pump and computer-readable storage medium.

### DESCRIPTION OF THE PRIOR ART

Lung cancer is one of the most common malignant tumors. In clinical treatment, surgical resection is still the first choice for the treatment of early lung cancer. However, lung cancer patients who are older, weaker, have poor cardiopulmonary function, or have complications, are unsuited to or intolerance of conventional surgical resection. Therefore, many local treatment methods such as minimally invasive tumor ablation have emerged.

Radio Frequency Ablation (RFA) is a relatively common minimally invasive tumor ablation treatment. The principle of radio frequency ablation is to apply alternating high-frequency current with a frequency less than 30MHz (megahertz) to make ions in tumor tissue oscillate at high speed of mutual friction, thereby converting radio frequency energy into heat energy, and causing coagulation necrosis of tumor cells.

During the ablation process, affected by the alternating high-frequency current, the temperature of the human body will change. Once the temperature exceeds the normal value, it will cause irreversible damage to the human body. Therefore, it is necessary to perfuse physiological saline into the ablation site during the ablation process. In the prior art, the perfusion of physiological saline is mainly done through a syringe pump. Before performing the perfusion, it is generally necessary to draw physiological saline into the syringe of the syringe pump. However, when drawing physiological saline, due to the lower pressure and lower gas solubility, bubble will occur in the syringe. In addition, if the physiological saline is drawn too soon, bubble will also occur. If the bubble enters the human body, it will cause harm to the human body. Therefore, how to discharge the bubble in the syringe in time is a major problem to be solved in the field.

### SUMMARY OF THE DISCLOSURE

### TECHNICAL PROBLEM

The embodiments of the present disclosure provide a control method and device for discharging bubble in a syringe pump, a syringe pump and a storage medium, which can realize automatic control of discharging bubble in a syringe pump, improve the timeliness of discharging bubble, and avoid the wasting of liquid in the syringe barrel and improve the efficiency of the bubble discharging operation.

### TECHNICAL SOLUTION

In one aspect, the embodiments of the present disclosure provide a control method for discharging bubble in a syringe pump, including:
Detecting whether there is a bubble in a syringe barrel of the syringe pump through a bubble detection device;
Determining a position of the bubble if there is a bubble in the syringe barrel; and
Controlling the syringe pump to perform a discharging operation according to the position of the bubble so as to discharge the bubble from the syringe barrel.

In one aspect, the embodiments of the present disclosure further provide a control device for discharging bubble in a syringe pump, including:
A detection module, configured to detect whether there is a bubble in a syringe barrel of the syringe pump through a bubble detection device;
A positioning module, configured to determine a position of the bubble if there is a bubble in the syringe barrel;
A control module, configured to control the syringe pump to perform a discharging operation according to the position of the bubble so as to discharge the bubble from the syringe barrel.

In one aspect, the embodiments of the present disclosure further provide an electronic device, including: a memory and a processor;
The memory stores executable program codes;
The processor coupled to the memory is configured to invoke the executable program codes stored in the memory to execute the control method for discharging bubble in a syringe pump as provided in the above embodiments.

In one aspect, the embodiments of the present disclosure further provide a syringe pump, including a controller, a bubble detection device, a syringe, a push-pull device and a driving device;
The controller is electrically coupled with the bubble detection device and the driving device;
The syringe includes a syringe barrel. One end of the push-pull device abuts against an end of the syringe, and the other end of the push-pull device is connected to the driving device;
The controller is configured to detect whether there is a bubble in the syringe barrel through the bubble detection device, determine a position of the bubble if there is a bubble in the syringe barrel, and control the push-pull device and the driving device to perform a discharging operation according to the position of the bubble so as to discharge the bubble from the syringe barrel.

In one aspect, the embodiments of the present disclosure further provide a computer-readable storage medium, in which a computer program is stored, wherein when the computer program is executed by a processor, the control method for discharging bubble in a syringe pump as provided in the above-mentioned embodiments is implemented.

### TECHNICAL ADVANTAGE

It can be seen from the above-mentioned embodiments of the present disclosure that, here, the bubble detection device is configured to detect whether there is a bubble in the syringe barrel of the syringe pump, when a bubble is detected in the syringe barrel, the position of the bubble is determined, and the syringe pump is controlled to perform a discharging operation according to the position of the bubble. In one aspect, the syringe pump can be controlled to automatically discharge the bubble in time, effectively avoiding the damage to the ablated object caused by the bubble during the perfusion operation, thereby improving the safety of the ablation operation. In another aspect, the discharging operation is performed according to the position of the bubble in the syringe barrel, that is, the discharging volume is determined according to the position of the bubble in the syringe barrel, avoiding the waste of liquid in the syringe barrel, and improving the efficiency of the bubble discharging operation.

### BRIEF DESCRIPTION OF DRAWINGS

In order to more clearly illustrate the technical solutions in the embodiments of the present disclosure or the prior art, the drawings will be briefly introduced in the following for describing the embodiments or the prior art. Apparently, the drawings in the following are only some embodiments of the present disclosure. For those skilled in the art, other drawings can be obtained according to these drawings without creative work.
FIG. 1 is a schematic diagram showing an application of a control method for discharging bubble in a syringe pump according to an embodiment of the present disclosure;
FIG. 2 is a schematic diagram of the internal structure of a syringe pump according to an embodiment of the present disclosure;
FIG. 3 is a schematic diagram of the internal structure of a syringe pump according to another embodiment of the present disclosure;
FIG. 4 is a flow chart of a control method for discharging bubble in a syringe pump according to an embodiment of the present disclosure;
FIG. 5 is a flow chart of a control method for discharging bubble in a syringe pump according to another embodiment of the present disclosure;
FIG. 6 is a schematic structural diagram of a control device for discharging bubble in a syringe pump according to an embodiment of the present disclosure;
FIG. 7 is a schematic structural diagram of a control device for discharging bubble in a syringe pump according to another embodiment of the present disclosure; and
FIG. 8 is a schematic structural diagram of hardware of an electronic device according to an embodiment of the present disclosure.

### DDESCRIPTION OF EMBODIMENTS

In order to make the purpose, technical solutions and advantages according to the embodiments of the disclosure apparent, the technical solutions according to the embodiments of the disclosure will be clearly and completely described below in conjunction with the drawings according to the embodiments of the disclosure. Apparently, the described embodiments are some of the embodiments of the present disclosure, not all of them. Based on the embodiments of the present disclosure, all other embodiments obtained by those skilled in the art without creative efforts fall within the protection scope of the present disclosure.

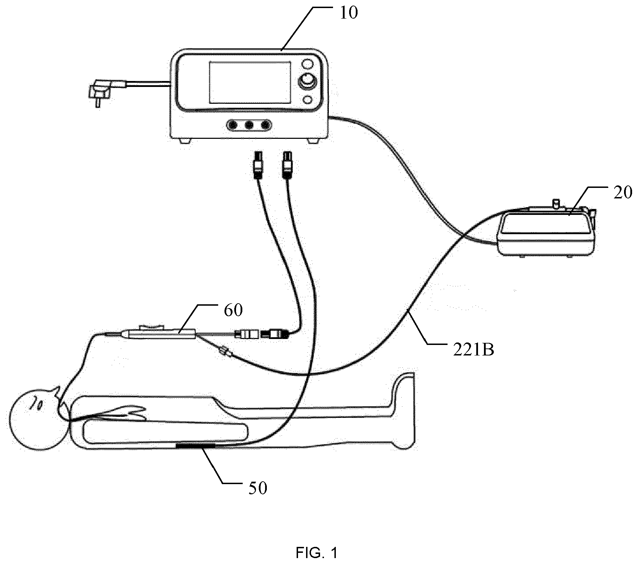

Referring to FIG. 1, a schematic diagram showing an application of a control method for discharging bubble in a syringe pump according to an embodiment of the present disclosure is shown. The control method for discharging bubble in a syringe pump can be implemented by the radio frequency ablation control device 10 or the syringe pump 20 as shown in FIG. 1. Alternatively, the control method for discharging bubble in a syringe pump can be implemented by other computer equipment other than the radio frequency ablation control device 10 or the syringe pump 20, such as: server, desktop computer, notebook computer, laptop computer, tablet computer, personal computer and smartphones etc.

In practical application, before performing the ablation, firstly, the bubble in the syringe barrel of the syringe pump is discharged by using the control method for discharging bubble in a syringe pump according to the following embodiments. Then, the radio frequency ablation catheter 60 for generating and outputting radio frequency energy and the extension tube 221B of the syringe pump 20 are inserted into the body of the object to be ablated (such as a lung cancer patient) and reach the ablation site. Thereafter, the neutral electrode 50 is brought into contact with the skin surface of the object to be ablated so that the radio frequency ablation catheter 60, the patient tissue and the neutral electrode 50 together form a loop when a radio frequency current is provided. The radio frequency ablation control device 10 controls the radio frequency ablation catheter 60 to output radio frequency energy to the ablation site by means of unipolar discharges, so as to perform ablation on the ablation site. At the same time, the syringe pump 20 performs perfusion of physiological saline to the ablation site to reduce the impedance and temperature of the ablation site, thereby reducing or avoiding the damage of the ablation to the ablation site.

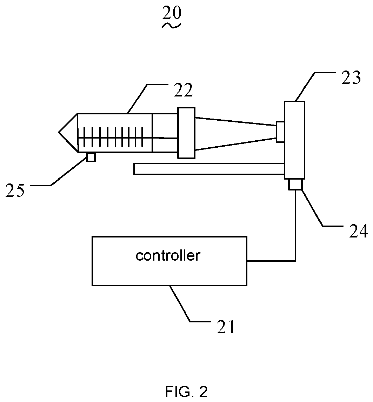

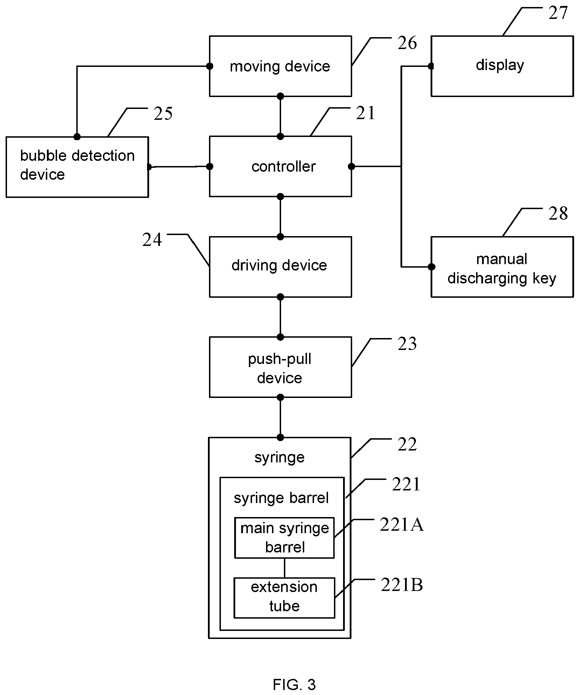

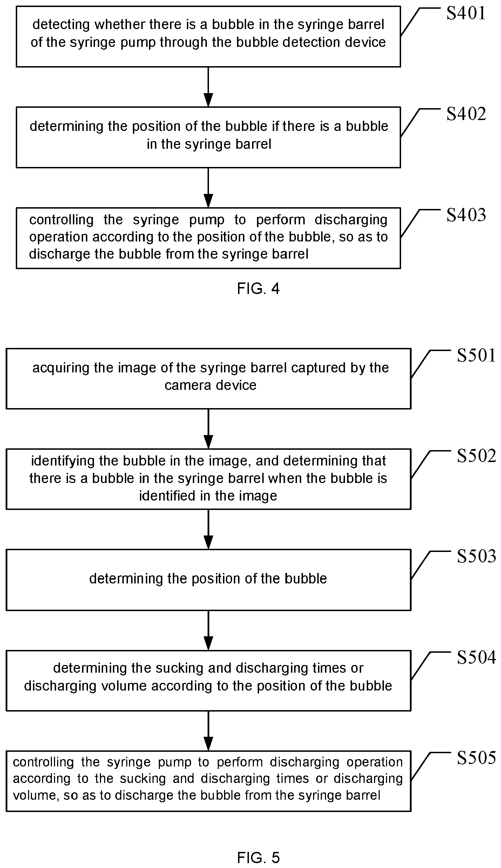

Referring to FIG. 2, a schematic diagram of the internal structure of the syringe pump according to an embodiment of the present disclosure is shown. As shown in FIG. 3, the syringe pump 20 includes a controller 21, a syringe 22, a push-pull device 23, a driving device 24 and a bubble detection device 25. By means of the controller 21, the control method for discharging bubble in a syringe pump according to the following embodiments shown in FIG. 4 and FIG. 5 can be implemented. For illustration, FIG. 2 only shows the parts associated with the embodiment of the present disclosure.

Specifically, the controller 21 is electrically coupled with the bubble detection device 25 and the driving device 24.

The syringe 22 includes a syringe barrel 221 (not denoted in FIG. 2). One end of the push-pull device 23 abuts against the end of the syringe 22, and the other end of the push-pull device 23 is connected to the driving device 24.

The driving device 24, such as various types of driving motors, is configured to, following the control instruction sent by the controller 21, drive the push-pull device 23 to move according to the speed indicated by the control instruction and the direction indicated by the control instruction, so as to push the fluid in the syringe barrel 221 into the ablation site or perform the discharging operation. The push-pull device 23 can be, for example, a push-pull rod.

The bubble detection device 25 may include, but is not limited to, a photoelectric sensor, an ultrasonic bubble detection sensor, and a camera. The bubble detection device 25 can be electrically coupled with the controller 21 through a data line or a built-in radio frequency transceiver for data interaction.

The controller 21 is configured to detect whether there is a bubble in the syringe barrel 221 through the bubble detection device 25, and if there is a bubble in the syringe barrel 221, then determine the position of the bubble, and perform the discharging operation through the push-pull device 23 and the driving device 24 according to the position of the bubble so as to discharge the bubble from the syringe barrel.

In the embodiment of the present disclosure, the bubble detection device is configured to detect whether there is a bubble in the syringe barrel of the syringe pump, when a bubble is detected in the syringe barrel, the position of the bubble is determined, and the syringe pump is controlled to perform the discharging operation according to the position of the bubble. In one aspect, the syringe pump can be controlled to automatically discharge the bubble in time, effectively avoiding the damage to the ablated object caused by the bubble during the perfusion operation, thereby improving the safety of the ablation operation. In another aspect, the discharging operation is performed according to the position of the bubble in the syringe barrel, that is, the discharging volume is determined according to the position of the bubble in the syringe barrel, avoiding the waste of liquid in the syringe barrel, and improving the efficiency of the bubble discharging operation.

Referring to FIG. 3, a schematic diagram of the internal structure of a syringe pump according to another embodiment of the present disclosure is shown. As shown in FIG. 3, different from the embodiment shown in FIG. 2, in this embodiment:
Optionally, the bubble detection device 25 is a camera device, such as a micro camera or a micro robot with a camera function. The syringe barrel 221 includes a main syringe barrel 221A and an extension tube 221B, and one end of the extension tube 221B is connected to the main syringe barrel 221A.

The controller 21 is further configured to acquire the image of the syringe barrel 221 captured by the camera device, and identify a bubble in the image, and if the bubble is identified in the image, it is determined that there is a bubble in the syringe barrel 221. The image captured by the camera device may be the image of the main syringe barrel 221A, or the image of the main syringe barrel 221A and the extension tube 221B.

Optionally, the bubble detection device 25 includes a plurality of camera devices, and the camera devices are respectively configured to capture images of different parts of the syringe barrel 221.

The controller 21 is further configured to, if there is a bubble in the syringe barrel 221, determine the target camera device that captured the image in which the bubble is identified, and determine the target part of the syringe barrel 221 corresponding to the target camera device according to the correspondence between the camera devices and different parts of the syringe barrel 221, and identify the target part as the position of the bubble.

Optionally, the syringe pump 20 further includes a housing (not shown in the figure) and a moving device 26.

Further, the moving device 26 includes a slide rail, a plurality of sliders and a driving motor (not shown in the figure). The slide rail is provided at a position corresponding to the main syringe barrel 221A of the syringe barrel 221 inside of the housing and corresponding to the extension tube 221B outside of the housing. The camera devices are connected to the sliders, and the driving motor is connected to the controller 21 and the sliders. The controller 21 controls the camera devices to move by instructing the driving motor to drive the sliders to move along the slide rail.

The controller 21 is further configured to acquire the size of the syringe barrel 221 and divide the syringe barrel 221 into a plurality of parts according to a preset division rule; determine the target positions and the target shooting parameters of the camera devices according to the size of the syringe barrel 221 and the positions of the divided parts; determine and record the correspondence between the camera devices and different parts of the syringe barrel 221 according to the target positions; and control the camera devices to move to the target positions, and adjust the shooting parameters of the camera devices to the target shooting parameters.

Optionally, the syringe pump 20 further includes a display 27 and a manual discharging key 28, and the display 27 and the manual discharging key 28 are electrically connected to the controller 21. The display 27 can be a touch panel or a non-touch panel.

The controller 21 is further configured to output a bubble discharging prompt message to the display 27 when it is detected that there is a bubble in the syringe barrel; and if an event that the user pressed the manual discharging key 28 is detected within a preset duration, the discharging operation is performed through the push-pull device 23 and the driving device 24.

Further, the syringe pump 20 further includes a power module (not shown in the figure), which is electrically connected to the controller 21, the driving device 24, the bubble detection device 25, the moving device 26 and the display 27, and is configured to provide power for the afore-mentioned devices.

Further, the syringe pump 20 further includes a network interface (not shown in the figure), the network interface is electrically connected to the controller 21, and the controller 21 establishes a data connection with the external devices through the network interface via a wireless or wired network for data interaction.

The details of this embodiment may refer to the relevant descriptions of the embodiments shown in FIG. 4 and FIG. 5 below.

In the embodiment of the present disclosure, the bubble detection device is configured to detect whether there is a bubble in the syringe barrel of the syringe pump, when a bubble is detected in the syringe barrel, the position of the bubble is determined, and the syringe pump is controlled to perform the discharging operation according to the position of the bubble. In one aspect, the syringe pump can be controlled to automatically discharge the bubble in time, effectively avoiding the damage to the ablated object caused by the bubble during the perfusion operation, thereby improving the safety of the ablation operation. In another aspect, the discharging operation is performed according to the position of the bubble in the syringe barrel, that is, the discharging volume is determined according to the position of the bubble in the syringe barrel, avoiding the waste of liquid in the syringe barrel, and improving the efficiency of the bubble discharging operation.

Referring to FIG. 4, a flow chart of a control method for discharging bubble in a syringe pump according to an embodiment of the present disclosure is shown. This method can be implemented by the syringe pump 20 shown in FIG. 1, or by the radio frequency ablation control device 10 shown in FIG. 1, or by other computer equipment electrically coupled to the syringe pump. As shown in FIG. 4, the method specifically includes:
Step S401, detecting whether there is a bubble in the syringe barrel of the syringe pump through the bubble detection device;
Specifically, the syringe pump includes a syringe, and the syringe includes a syringe barrel for containing physiological saline. At least one bubble detection device is arranged on the syringe barrel or near the syringe barrel, so as to detect whether there is a bubble in the syringe barrel through the bubble detection device. The bubble detection device may include, but is not limited to, a photoelectric sensor, an ultrasonic bubble detection sensor, and a camera.

If a plurality of bubble detection devices is provided, the bubble detection devices can be evenly distributed on or near different parts of the syringe barrel, for example, on two ends and the middle of the syringe barrel, or evenly distributed on the whole outside wall of the syringe barrel.

Step S402, determining the position of the bubble if there is a bubble in the syringe barrel;
Specifically, if there is a bubble in the syringe barrel, the position of the bubble in the syringe barrel can be determined according to, for example, the position of the bubble detection device, the direction of the feedback signal received by the bubble detection device for determining the presence of bubble, or the position of the bubble in the image captured by the bubble detection device.

Further, if there is no bubble in the syringe barrel, the syringe pump is controlled to perform the perfusion operation. Further, before controlling the syringe pump to perform the perfusion operation, a prompt message can be output to remind the user that there is no bubble in the syringe barrel, and ask the user to confirm whether or not to control the syringe pump to perform the perfusion operation. Then, according to the control instruction for performing perfusion operation triggered by the user based on the prompt message, the syringe pump is controlled to perform the perfusion operation.

By outputting the prompt message and performing the perfusion operation according to the control instruction triggered by the user, the user can be reminded timely to perform the perfusion operation, improving the controllability of the perfusion operation.

Step S403, controlling the syringe pump to perform the discharging operation according to the position of the bubble, so as to discharge the bubble from the syringe barrel.

Specifically, the discharging volume can be determined according to the position of the bubble in the syringe barrel, or the discharging times can be determined according to a preset discharging volume. Then, according to the determined discharging volume or times, the syringe pump is controlled to perform the discharging operation, so as to discharge the bubble from the syringe barrel. The discharging volume refers to the amount of liquid that needs to be discharged.

Optionally, in another embodiment of the present disclosure, the method is implemented by the radio frequency ablation control device 10 shown in FIG. 1 or other computer equipment electrically coupled to the syringe pump. Before detecting whether there is a bubble in the syringe barrel of the syringe pump through the bubble detection device, the method further includes:
Checking whether the connection with the syringe pump is normal;
Controlling the syringe pump to perform the discharging operation according to the preset discharging volume and following the triggered discharging instruction when it is detected that the connection with the syringe pump is normal.

The discharging instruction can be automatically triggered when it is detected that the connection with the syringe pump is normal. Alternatively, the discharging instruction can be triggered according to a preset operation by the user, for example, an operation of pressing the physically manual discharging key by the user, or an operation of clicking a preset virtual discharging control button on the discharging control interface by the user.

Specifically, for example, it may be detected whether the connection with the syringe pump is normal by sending a detection signal to the syringe pump. On the one hand, if the syringe pump feeds back a response signal within a preset duration, it can be determined that the connection with the syringe pump is normal, and the discharging instruction is automatically triggered, and then according to the discharging instruction, the syringe pump is controlled to perform the discharging operation once according to the preset discharging volume. Alternatively, when it is determined that the connection with the syringe pump is normal, a prompt message can be output, and then, according to the discharging instruction triggered by the user based on the prompt message, the syringe pump is controlled to perform a manual discharging operation according to the preset discharging volume.

On the other hand, if the syringe pump does not feedback a response signal within a preset duration, it can be determined that the connection with the syringe pump is abnormal, and a prompt message is output to remind the user that the connection is abnormal.

By controlling the syringe pump to perform the discharging operation first when it is detected that the connection with the syringe pump is normal, the timeliness and the success rate of bubble discharging can be further improved. Further, after controlling the syringe pump to perform the discharging operation first, the syringe pump can be controlled to perform the discharging operation again according to the bubble detection result, which can further ensure that the bubble is completely discharged, thereby further improving the efficiency of the discharging operation.

Optionally, in order to further improve the controllability and flexibility of the discharging operation, in another embodiment of the present disclosure, before step S403 of controlling the syringe pump to perform the discharging operation according to the position of the bubble, the method may further include the following steps:
Outputting a bubble discharging prompt message;
If an event that the user pressed the manual discharging key is detected within a preset duration, the syringe pump is controlled to perform the discharging operation according to the discharging times and the discharging volume indicated by the event;
If the event that the user pressed the manual discharging key is not detected within a preset duration, the syringe pump is controlled to perform the discharging operation according to the position of the bubble, so as to discharge the bubble from the syringe barrel of the syringe pump.

In the embodiment of the present disclosure, the bubble detection device is configured to detect whether there is a bubble in the syringe barrel of the syringe pump, when a bubble is detected in the syringe barrel, the position of the bubble is determined, and the syringe pump is controlled to perform the discharging operation according to the position of the bubble. In one aspect, the syringe pump can be controlled to automatically discharge the bubble in time, effectively avoiding the damage to the ablated object caused by the bubble during the perfusion operation, thereby improving the safety of the ablation operation. In another aspect, the discharging operation is performed according to the position of the bubble in the syringe barrel, that is, the discharging volume is determined according to the position of the bubble in the syringe barrel, avoiding the waste of liquid in the syringe barrel, and improving the efficiency of the bubble discharging operation.

Referring to FIG. 5, a flow chart of a control method for discharging bubble in a syringe pump according to another embodiment of the present disclosure is shown. This method can be implemented by the syringe pump 20 shown in FIG. 1, or by the radio frequency ablation control device 10 shown in FIG. 1, or by other computer equipment electrically coupled to the syringe pump. As shown in FIG. 5, the method specifically includes:
Step S501, acquiring the image of the syringe barrel captured by the camera device;
Optionally, the syringe barrel includes a main syringe barrel and an extension tube. The camera device may include, but is not limited to, a micro camera, or a micro robot with a camera function.

In the present disclosure, at least one camera device can be provided. The afore-mentioned at least one camera device can be used to shoot the complete syringe barrel or a designated part of the syringe barrel in real time or periodically or before performing the perfusion, and the image fed back by the camera device can be acquired. The image can be a static picture or a dynamic image.

Step S502, identifying the bubble in the image, and determining that there is a bubble in the syringe barrel when a bubble is identified in the image;
Specifically, image identification technologies can be used to identify the bubble in the image captured by the camera device. For example, at first, filtering methods such as mean filtering, Wiener filtering, adaptive median filtering, etc. can be used to eliminate noise in the image; then, at least one of Sobel operator, Laplance operator, Canny operator, etc., for example, is used to detect the edge of the filtered image; finally, according to the edge detection result, it can be determined whether there is a bubble in the image. Among them, the edge of the image refers to a collection of points with discontinuous or drastically varied image gray values, such as the boundaries of the bubble and the syringe barrel. Optionally, when more than two operators are used for edge detection, all detection results can be added together so as to identify the bubble in the image more accurately.

Step S503, determining the position of the bubble;
Optionally, in another embodiment of the present disclosure, the position of the bubble can be specifically determined through the following steps:
Determining the position of the bubble in the image if there is a bubble in the syringe barrel; and
Determining the position of the bubble in the syringe barrel according to the position of the bubble in the image and the mapping relationship between the syringe barrel in the image and the actual syringe barrel.

Specifically, while identifying the bubble, the position of the bubble in the syringe barrel in the image is also determined. Then, according to the position of the bubble in the syringe barrel in the image, and the mapping relationship between the syringe barrel in the preset image and the actual syringe barrel, the position of the bubble in the actual syringe barrel is determined.

Among them, the mapping relationship between the syringe barrel in the preset image and the actual syringe barrel refers to the correspondence between the coordinates of the feature points on the syringe barrel in the image and the coordinates of the feature points on the actual syringe barrel.

It can be understood that when a plurality of camera devices are used for shooting, the captured images can be combined first to obtain an image including the complete syringe barrel, and then the bubble and the position thereof in the image can be determined.

If the combination of the parts captured by the camera devices is not a complete syringe barrel, when combining the images captured, preset description parameters of the part of the syringe barrel that cannot be shot by the camera devices are used to draw the un-captured part. Then, the drawn image is inserted into the corresponding position in the image obtained by combining the captured images, so as to obtain an image including the complete syringe barrel. The description parameters may specifically include, but are not limited to, position and size (such as inner diameter, outer diameter, wall thickness, length, width, and height) and the like.

Further, before step S501 of detecting whether there is a bubble in the syringe barrel of the syringe pump through the bubble detection device, the method may further include:
Acquiring the size of the syringe barrel, and adjusting the shooting parameters of the camera device according to the size of the syringe barrel.

Specifically, the models of the syringe barrels input by the user when setting the perfusion operation can be acquired first, and then the size of the current syringe barrel can be determined according to the correspondence between the preset syringe barrel models and the respective syringe barrel sizes, and according to the size, the target shooting parameters of the camera devices can be determined, and the respective shooting parameters of the camera devices are adjust to the target shooting parameters. The shooting parameters may include, but are not limited to, shooting angle, focal length, pixel, minimum illuminance, and sensitivity. The size of the syringe barrel includes length, width, height, wall thickness, etc.

By adjusting the shooting parameters of the camera devices according to the size of the syringe barrel before shooting, it can be ensured that the camera devices can accurately capture the corresponding images regardless of the size of the syringe barrel, thereby further improving the efficiency of the discharging operation, and also omitting the step of manually adjusting the shooting parameters, thereby improving the intelligence of the discharging operation.

Optionally, in another embodiment of the present disclosure, when a plurality of camera devices are used as the bubble detection device, and the a plurality of camera devices are respectively located for shooting different parts of the syringe barrel, the position of the bubble can be determined by the following steps:
Determining the position of the target camera device that captured the image in which the bubble is identified if there is a bubble in the syringe barrel; and
Determining the target part of the syringe barrel corresponding to the target camera device according to the correspondence between the camera devices and different parts of the syringe barrel, and identifying the target part as the position of the bubble.

Specifically, the camera devices send the respective identification information while sending the captured images. According to the preset correspondence between the identification information of the camera devices and the identification information of different parts of the syringe barrel, the target part of the syringe barrel corresponding to the target camera device can be determined, and the position of the target part of the syringe barrel is the position of the bubble in the syringe barrel.

Determining the position of the bubble according to the identification of the camera device can improve the calculation speed since the calculation amount is small, reducing the resource occupation.

Further, before detecting whether there is a bubble in the syringe barrel of the syringe pump through the bubble detection device, the method further includes the following steps:
Determining the target positions and target shooting parameters of the camera devices according to the size of the syringe barrel and the positions of the divided parts;
Determining and recording the correspondence between the camera devices and different parts of the syringe barrel according to the target positions of the camera devices; and
Controlling the camera devices to move to the target positions, and adjusting the shooting parameters of the camera devices to the target shooting parameters.

Specifically, the division rule may include, but is not limited to, at least one of the number of divided parts, division positions, division starting position, division ending position, and the sizes of the divided parts.

According to the installation position of the syringe barrel inside and / or outside the syringe pump, as well as the size of the syringe barrel and the division rule, the positions of the divided parts can be determined. The afore-mentioned positions can be represented in coordinates. The coordinate system of the coordinates is a two-dimensional or three-dimensional coordinate system established with the center of mass of the syringe pump as the origin. Provided that the syringe barrel is installed inside the syringe pump and the syringe barrel is evenly divided into 3 parts, according to the initial coordinate of the installation position of the syringe barrel and the size of the syringe barrel, the coordinates of the front end and the rear end of the syringe barrel can be determined, and then according to the size of each part of the syringe barrel and the even division rule, the coordinates of the front end and the rear end of each part of the syringe barrel can be determined. Then, the resulted coordinate of the front end or the rear end or the median between the front end and the rear end of each part of the syringe barrel can be selected as the coordinate of the target position of each camera device and the identification information of the corresponding part. The camera devices are assigned with identification information, and then the identification information of the camera devices are associated with the coordinates of the respective target positions, and a correspondence between the camera devices and the respective parts of the syringe barrel is generated. At the same time, according to the coordinates of the target positions, the respective shooting parameters are determined for the camera devices, so that the camera devices can clearly capture the respective parts.

The camera devices can be assigned with respective target position coordinates randomly. Alternatively, the camera devices can be assigned with respective target position coordinates according to the current position and target position coordinates of the camera devices following the principle of the shortest moving route.

Further, the syringe barrel includes a main syringe barrel and an extension tube. The main syringe barrel is generally arranged inside the syringe pump, and the extension tube is partly arranged outside the syringe pump. One end of the extension tube is connected with the main syringe barrel. The length of the main syringe barrel can be determined according to the model of the main syringe barrel, and the length of the extension tube is a preset value. According to the coordinate of the connection between the main syringe barrel and the extension tube, and the preset length of the extension tube, the coordinate of the free end of the extension tube (the end inserted into the ablation site) can be determined.

Optionally, different shooting parameter configuration rules can be provided for different positions to adapt to different lighting conditions inside and outside the syringe pump. Specifically, it can be determined according to the coordinates of the target positions that whether the corresponding target positions are inside or outside the syringe pump, then the corresponding shooting parameter configuration rules are acquired according to the determination result, and then the target shooting parameters of the camera devices are determined according to the coordinates of the target positions and the corresponding shooting parameter configuration rules.

Providing different shooting parameter configuration rules for different positions can improve the clarity and integrity of the images captured by the camera devices, thereby reducing the calculation difficulty of bubble identification, and improving the accuracy and speed of bubble identification.
Step S504, determining the sucking and discharging times or discharging volume according to the position of the bubble;
Step S505, controlling the syringe pump to perform the discharging operation according to the sucking and discharging times or discharging volume, so as to discharge the bubble from the syringe barrel.

Specifically, the discharging volume (that is, the amount of liquid that needs to be discharged so that the bubble can be completely discharged) can be determined according to the position of the bubble in the syringe barrel.

The sucking and discharging times include sucking times and discharging times, and the amount of liquid sucked or discharged each time is a preset value. When the bubble is close to the rear end of the syringe barrel, one discharging operation may not be able to completely discharge the bubble. In such case, a plurality of sucking and discharging operations can ensure that the bubble is effectively discharged, while there is still enough liquid remained in the syringe barrel, thereby improving efficiency of the discharging operation.

Optionally, in order to improve the controllability and flexibility of the discharging operation, in another embodiment of the present disclosure, before step S504 of determining the sucking and discharging times or the discharging volume according to the position of the bubble, the method may further include the following steps:
Outputting a bubble discharging prompt message;
If an event that the user pressed the manual discharging key is detected within a preset duration, the syringe pump is controlled to perform the discharging operation according to the discharging times or the discharging volume indicated by the event; and
If the event that the user pressed the manual discharging key is not detected within a preset duration, the step of determining the position of the bubble is performed.

In other words, when a bubble is detected in the syringe barrel, the bubble discharging prompt message is firstly output to remind the user that there is a bubble in the syringe barrel as well as the location of the bubble, and ask the user to confirm whether or not to perform manual discharging operation; then, when the event that the user pressed the manual discharging key is detected by the event monitor within a preset duration, it is determined that the event that the user pressed the manual discharging key is detected, and the syringe pump is controlled to perform the discharging operation according to the discharging times or the discharging volume indicated by the event. The discharging times or the discharging volume indicated by the event that the user pressed the manual discharging key can be set as user defined. For example, when the user pressed the manual discharging key once, the discharging operation will be performed once, and the discharging volume is 10 milliliters.

In another aspect, if the event monitor does not detect the occurrence of an event that the user pressed the manual discharging key within a preset duration, steps S504 and S505 are performed to perform an automatic discharging operation.

The bubble discharging prompt message can be output in the form of at least one of text, image, warning light, and prompt sound.

Optionally, the method further includes controlling the syringe pump to stop the discharging operation according to a stopping control instruction triggered by the user at any time point between the output of the bubble discharging prompt message and the completion of the discharging operation.

Stopping the discharging operation means that if the discharging operation has not been performed, the action is canceled, and if the discharging operation has been performed, the action is stopped. The stopping control instruction can be triggered according to the operation of the user pressing a physical button or a virtual button for triggering the instruction.

By combining manual discharging and automatic discharging through outputting a bubble discharging prompt message, the controllability and flexibility of the discharging operation can be further improved.

In the embodiment of the present disclosure, the bubble detection device is configured to detect whether there is a bubble in the syringe barrel of the syringe pump, when a bubble is detected in the syringe barrel, the position of the bubble is determined, and the syringe pump is controlled to perform the discharging operation according to the position of the bubble. In one aspect, the syringe pump can be controlled to automatically discharge the bubble in time, effectively avoiding the damage to the ablated object caused by the bubble during the perfusion operation, thereby improving the safety of the ablation operation. In another aspect, the discharging operation is performed according to the position of the bubble in the syringe barrel, that is, the discharging volume is determined according to the position of the bubble in the syringe barrel, avoiding the waste of liquid in the syringe barrel, and improving the efficiency of the bubble discharging operation.

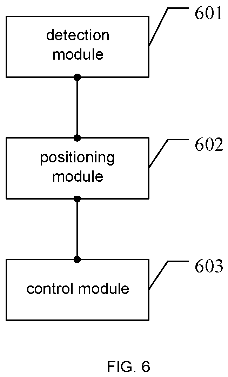

Referring to FIG. 6, a schematic structural diagram of a control device for discharging bubble in a syringe pump according to an embodiment of the present disclosure is disclosed. For illustration, FIG. 6 only shows the parts associated with the embodiment of the present disclosure. The device can be arranged in the syringe pump 20 or the radio frequency ablation control device 10 shown in FIG. 1, or can be arranged in other computer equipment. The device includes a detection module 601, a positioning module 602 and a control module 603.

The detection module 601 is configured to detect whether there is a bubble in the syringe barrel of the syringe pump through the bubble detection device;
The positioning module 602 is configured to determine the position of the bubble if there is a bubble in the syringe barrel;
The control module 603 is configured to control the syringe pump to perform the discharging operation according to the position of the bubble, so as to discharge the bubble from the syringe barrel.

The specific process for realizing the respective functions of the above-mentioned modules can refer to the embodiments shown in FIG. 4 and FIG. 5, which will not be repeated here.

In the embodiment of the present disclosure, the bubble detection device is configured to detect whether there is a bubble in the syringe barrel of the syringe pump, when a bubble is detected in the syringe barrel, the position of the bubble is determined, and the syringe pump is controlled to perform the discharging operation according to the position of the bubble. In one aspect, the syringe pump can be controlled to automatically discharge the bubble in time, effectively avoiding the damage to the ablated object caused by the bubble during the perfusion operation, thereby improving the safety of the ablation operation. In another aspect, the discharging operation is performed according to the position of the bubble in the syringe barrel, that is, the discharging volume is determined according to the position of the bubble in the syringe barrel, avoiding the waste of liquid in the syringe barrel, and improving the efficiency of the bubble discharging operation.

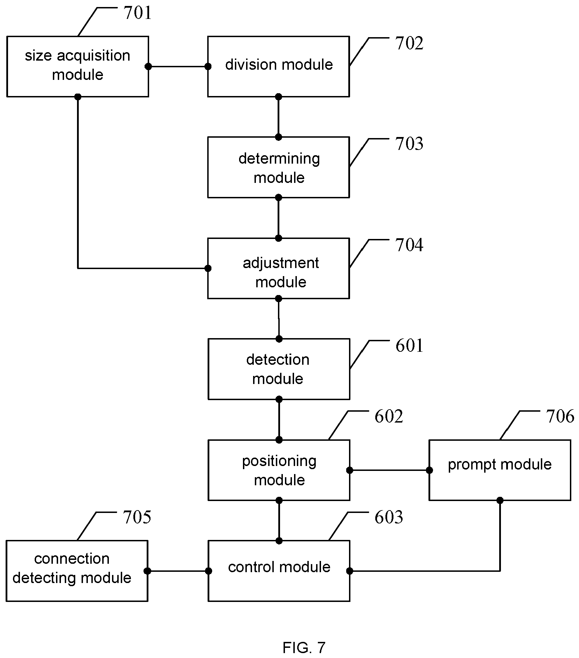

Referring to FIG. 7, a schematic structural diagram of a control device for discharging bubble in a syringe pump according to another embodiment of the present disclosure is shown. For illustration, FIG. 7 only shows the parts associated with the embodiment of the present disclosure. The device can be arranged in the syringe pump 20 or the radio frequency ablation control device 10 shown in FIG. 1, or can be arranged in other computer equipment. This embodiment differs from the embodiment shown in FIG. 6 in the followings.

Further, the bubble detection device is a camera, and the detection module 601 is further configured to acquire the image of the syringe barrel captured by the camera and determine that there is a bubble detected in the syringe barrel if the bubble is identified in the image.

Further, the positioning module 602 is further configured to determine the position of the bubble if there is a bubble in the syringe barrel in the image and determine the position of the bubble in the syringe barrel according to the position of the bubble in the image and the mapping relationship between the syringe barrel in the image and the actual syringe barrel.

Further, the bubble detection device includes a plurality of camera devices for shooting different parts of the syringe barrel. The positioning module 602 is further configured to determine the position of the target camera device that captured the image in which the bubble is identified if there is a bubble in the syringe barrel, and determine the target part of the syringe barrel corresponding to the target camera device according to the correspondence between the camera devices and different parts of the syringe barrel, and identify the target part as the position of the bubble.

Further, the device further includes:
A size acquisition module 701, configured to acquire the size of the syringe barrel;
A division module 702, configured to divide the syringe barrel into a plurality of parts according to a preset division rule;
A determining module 703, configured to determine the target positions and target shooting parameters of the camera devices according to the size of the syringe barrel and the positions of the divided parts; and determine and record the correspondence between the camera devices and different parts of the syringe barrel 221 according to the target positions; and
An adjustment module 704, configured to control the camera devices to move to the target positions, and adjust the shooting parameters of the camera devices to the target shooting parameters.

Further, the control module 603 is further configured to determine the sucking and discharging times or discharging volume according to the position of the bubble; and control the syringe pump to perform the discharging operation according to the sucking and discharging times or the discharging volume.

Further, the adjustment module 704 is further configured to adjust the shooting parameters of the camera devices according to the size of the syringe barrel.

Further, the device further includes:
A connection detecting module 705, configured to detect whether the connection with the syringe pump is normal;
The control module 603 is further configured to control the syringe pump to perform the discharging operation according to the preset discharging volume and following the triggered discharging instruction when the connection detecting module 705 detects that the connection with the syringe pump is normal.

Further, the device further includes:
A prompt module 706, configured to output a bubble discharging prompt message;
The control module 603 is further configured to control the syringe pump to perform the discharging operation according to the discharging times or the discharging volume indicated by the event if the event that the user pressed the manual discharging key is detected within a preset duration;

The control module 603 is further configured to control the syringe pump to perform the discharging operation according to the position of the bubble if the event is not detected within a preset duration, so as to discharge the bubble from the syringe barrel of the syringe pump.

The specific process for realizing the respective functions of the above-mentioned modules can refer to the embodiments shown in FIG. 4 and FIG. 5, which will not be repeated here.

In the embodiment of the present disclosure, the bubble detection device is configured to detect whether there is a bubble in the syringe barrel of the syringe pump, when a bubble is detected in the syringe barrel, the position of the bubble is determined, and the syringe pump is controlled to perform the discharging operation according to the position of the bubble. In one aspect, the syringe pump can be controlled to automatically discharge the bubble in time, effectively avoiding the damage to the ablated object caused by the bubble during the perfusion operation, thereby improving the safety of the ablation operation. In another aspect, the discharging operation is performed according to the position of the bubble in the syringe barrel, that is, the discharging volume is determined according to the position of the bubble in the syringe barrel, avoiding the waste of liquid in the syringe barrel, and improving the efficiency of the bubble discharging operation.

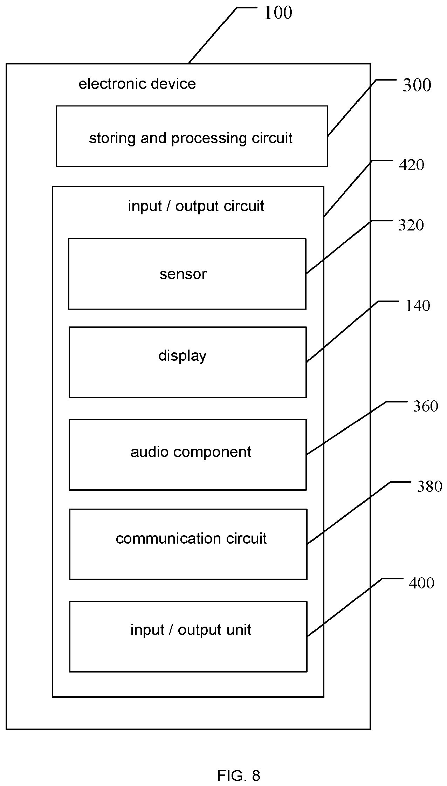

Referring to FIG. 8, a schematic structural diagram of hardware of an electronic device according to an embodiment of the present disclosure is shown.

Exemplarily, the electronic device may be any of various types of non-removable or removable or portable computer system devices performing wireless or wired communications. Specifically, the electronic device may be a desktop computer, a server, a mobile phone or a smart phone (for example, a phone based on iPhone TM or Android TM); a portable game device (for example, Nintendo DS TM, PlayStation Portable TM, Gameboy Advance TM, iPhone TM), a laptop, a PDA, a portable Internet device, a portable medical device, a smart camera, a music player, and a data storage device; other handheld device such as watch, earphones, pendant, headphone; or other wearable device such as electronic glasses, electronic cloth, electronic bracelet, electronic necklace, and other head-mounted device ( HMD).

In some cases, the electronic device can perform various functions, for example, playing music, displaying video, storing images, and receiving and sending phone calls.

As shown in FIG. 8, the electronic device 100 may include a control circuit, which may include a storing and processing circuit 300. The storing and processing circuit 300 may include a memory, such as hard disk drive memory, non-volatile memory (such as flash memory or other electronically programmable limited-erasable memory for forming solid-state drive, etc.), volatile memory (such as static or dynamic random access memory, etc.), and the like, which is not limited in this embodiment of the present disclosure. The processing circuit in the storing and processing circuit 300 may be configured to control the operation of the electronic device 100. The processing circuit may be embodied based on one or more microprocessors, microcontrollers, digital signal processors, baseband processors, power management units, audio codec chips, application specific integrated circuits, display driver integrated circuits, and the like.

The storing and processing circuit 300 can be configured to run software in the electronic device 100, such as Internet browsing applications, Voice over Internet Protocol (VOIP) telephone call applications, email applications, media player applications, operating system functions, etc. The software can be configured to perform control operations, such as image acquisition based on camera, ambient light measurement based on ambient light sensor, proximity sensor measurement based on proximity sensor, information display based on status indicators such as LEDs, touch event detection based on touch sensor, information display on a plurality of (e.g. layered) displayers, operations related to wireless communication, operations related to audio signal collection and generation, control operations related to data collection and process of button press event, and other functions in the electronic device 100, which are not limited in the present embodiment of the present disclosure.

Further, the memory stores executable program codes, and the processor coupled to the memory is configured to invoke the executable program codes stored in the memory to execute the control method for discharging bubble in a syringe pump described in the embodiments referring to FIG. 4 and FIG. 5 above.

The executable program codes include various modules provided in the control device for discharging bubble in a syringe pump described in the above-mentioned embodiments shown in FIG. 6 and FIG. 7, such as the detection module 601, the positioning module 602 and the control module 603. The specific process for realizing the respective functions of the above-mentioned modules can refer to the embodiments shown in FIG. 4 through FIG. 7, which will not be repeated here.

The electronic device 100 may further include an input / output circuit 420. The input / output circuit 420 can be configured to enable the electronic device 100 to realize data input and output, that is, allow the electronic device 100 to receive data from external devices and also allow the electronic device 100 to output data from the electronic device 100 to external devices. The input / output circuit 420 may further include a sensor 320. The sensor 320 may be an ambient light sensor, a proximity sensor based on light and capacitance, a touch sensor (for example, a light and/ or capacitance based touch sensor which can be provided as a part of the touch panel or used independently), acceleration sensor, or other sensors, etc.

The input / output circuit 420 may further include one or more displays, such as display 140. The display 140 may include one or more of liquid crystal display, organic light emitting diode display, electronic ink display, plasma display, and displays using other display technologies. The display 140 may include a touch sensor array, that is, the display 140 may be a touch panel. The touch sensor may be a capacitive touch sensor formed from an array of transparent touch sensor electrodes such as indium tin oxide (ITO) electrodes, or may be a touch sensor formed using other touch technologies, such as acoustic touch, pressure sensitive touch, resistive touch, optical touch, etc., which are not limited in the present embodiment of the disclosure.

The electronic device 100 may further include an audio component 360. The audio component 360 may be configured to provide audio input and output for the electronic device 100. The audio component 360 in the electronic device 100 may include speaker, microphone, buzzer, tone generator and other components for generating and detecting sounds.

A communication circuit 380 can be configured to provide the electronic device 100 with the ability to communicate with external devices. The communication circuit 380 may include an analog and digital input / output interface circuit, and a wireless communication circuit based on radio frequency signals and / or optical signals. The wireless communication circuit in the communication circuit 380 may include a radio frequency transceiver circuit, a power amplifier circuit, a low noise amplifier, a switch, a filter, and an antenna. For example, the wireless communication circuit in the communication circuit 380 may include a circuit for supporting Near Field Communication (NFC) by transmitting and receiving near field coupled electromagnetic signals. For example, the communication circuit 380 may include a near field communication antenna and a near field communication transceiver. The communication circuit 380 may further include a cellular telephone transceiver and antennas, a wireless local area network transceiver circuit and antennas, and the like.

The electronic device 100 may further include a battery, a power management circuit and other input / output unit 400. The input / output unit 400 may include a button, joystick, click wheel, scroll wheel, touch pad, keypad, keyboard, camera, light emitting diode and other status indicators, and the like.

The user can input instructions through the input / output circuit 420 to control the operation of the electronic device 100, and can receive status information and other outputs from the electronic device 100 through the output data of the input / output circuit 420.

Further, the embodiment of the present disclosure provides a computer-readable storage medium, which can be arranged in the electronic device according to the above-mentioned embodiments, and the computer-readable storage medium can be the memory in the storing and processing circuit 300 described in the embodiment referring to FIG. 8. The computer-readable storage medium stores a computer program through which the control method for discharging bubble in a syringe pump according to the embodiments shown in FIG. 4 and FIG. 5 can be implemented when executed by the processor. The specific process for realizing the control method for discharging bubble in a syringe pump above can refer to the embodiments shown in FIG. 4 and FIG. 5, which will not be repeated here.

Further, the computer storage medium may be a USB disk, a mobile hard disk, a Read-Only Memory (ROM), RAM, a magnetic disk or an optical disk, or other mediums that can store program codes.

In the several embodiments provided in this disclosure, it should be understood that the disclosed devices and methods may be implemented in other manners. For example, the devices according to the embodiments described above are only illustrative. For example, the division of the modules is only a division from the perspective of logical function, and in practice, other division manners can be used. For example, a plurality of modules or components can be combined or integrated into another system, or some features may be omitted or don't work. In another aspect, the mutual coupling or direct coupling or communication connection shown or discussed may be indirect coupling or communication connection between devices or modules via some interfaces, in electrical, mechanical or other forms.

The modules described as separate components may or may not be physically separated, and the components displayed as modules may or may not be physical modules, that is, they may be located in one place, or may be distributed on a plurality of network modules. Part or all of the modules can be used according to practical needs to achieve the purposes of the solutions of the embodiments.

In addition, the functional modules in each embodiment of the present disclosure may be integrated into one processing module, or may present separately physically, or two or more modules may be integrated into one module. The above-mentioned integrated modules can be implemented in the form of hardware or in the form of software modules.

If the integrated modules are implemented in the form of software modules and sold or used as independent products, they can be stored in a computer-readable storage medium. In view of this, the technical solution of the present disclosure or the part that contributes to the prior art or all or part of the technical solution can be embodied in the form of software which can be stored in a readable medium, including several instructions to enable a computer device (which may be a personal computer, server, or network device, etc.) to execute all or part of the steps of the methods described in the various embodiments of the present disclosure. The aforementioned readable storage medium includes various mediums capable of storing program codes, such as USB disk, mobile hard disk, ROM, RAM, magnetic disk or optical disk.

It should be noted that, for the sake of simplicity of description, the aforementioned method embodiments are described as combinations of serious of steps, but those skilled in the art should know that the present disclosure is not limited by the described step sequences. According to the disclosure, some steps may be performed in other orders or simultaneously. Further, those skilled in the art should also know that the embodiments described in the description are all preferred embodiments, and the steps and modules involved are not necessarily required.

The above embodiments are described from different aspects, and aspects of some embodiments that are not described in detail can refer to relevant aspects of other embodiments.

The above describes the control method for discharging bubble in a syringe pump, device, syringe pump and computer-readable storage medium disclosed here. For those skilled in the art, based on the concept of the embodiments of the present disclosure, the specific implementation and application may change. In summary, the content of this description should not be interpreted as limitation to the present invention.

## Claims

1. A control method for discharging bubble in a syringe pump, comprising steps of:
detecting whether there is a bubble in a syringe barrel of the syringe pump through a bubble detection device;
determining a position of the bubble if there is a bubble in the syringe barrel; and
controlling the syringe pump to perform a discharging operation according to the position of the bubble so as to discharge the bubble from the syringe barrel.

2. The method according to claim 1, wherein the bubble detection device comprises a camera device, and the step of detecting whether there is a bubble in the syringe barrel of the syringe pump through the bubble detection device comprises:
acquiring an image of the syringe barrel captured by the camera device; and
identifying a bubble in the image, and determining that there is a bubble in the syringe barrel if a bubble is identified in the image.

3. The method according to claim 2, wherein the step of determining the position of the bubble if there is a bubble in the syringe barrel comprises:
determining a position of the bubble in the image if there is a bubble in the syringe barrel; and
determining the position of the bubble in the syringe barrel according to the position of the bubble in the image and a mapping relationship between the syringe barrel in the image and the syringe barrel.

4. The method according to claim 2, wherein the bubble detection device comprises a plurality of camera devices respectively configured to shoot different parts of the syringe barrel, and the step of determining the position of the bubble if there is a bubble in the syringe barrel comprises:
determining the target camera device that captured the image in which the bubble is identified if there is a bubble in the syringe barrel; and
determining a target part of the syringe barrel corresponding to the target camera device as the position of the bubble according to a correspondence between the camera devices and different parts of the syringe barrel.

5. The method according to claim 4, further comprising, before the step of detecting whether there is a bubble in the syringe barrel of the syringe pump through the bubble detection device:
acquiring a size of the syringe barrel, and dividing the syringe barrel into a plurality of parts according to a preset division rule;
determining target positions and target shooting parameters of the camera devices according to the size of the syringe barrel and positions of the divided parts;
determining and recording the correspondence between the camera devices and the different parts of the syringe barrel according to the target positions; and
controlling the camera devices to move to the target positions, and adjusting shooting parameters of the camera devices to the target shooting parameters.

6. The method according to claim 2, further comprising, before the step of detecting whether there is a bubble in the syringe barrel of the syringe pump through the bubble detection device:
acquiring a size of the syringe barrel, and adjusting shooting parameters of the camera device according to the size of the syringe barrel.

7. The method according to any one of claims 1 to 6, wherein the step of controlling the syringe pump to perform a discharging operation according to the position of the bubble comprises:
determining sucking and discharging times or discharging volume according to the position of the bubble; and
controlling the syringe pump to perform the discharging operation according to the sucking and discharging times or the discharging volume.

8. The method according to any one of claims 1 to 6, further comprising, before the step of detecting whether there is a bubble in the syringe barrel of the syringe pump through the bubble detection device:
detecting whether a connection with the syringe pump is normal; and
controlling the syringe pump to perform the discharging operation following a triggered discharging instruction and according to a preset discharging volume when it is detected that the connection with the syringe pump is normal.

9. The method according to any one of claims 1 to 6, further comprising, before controlling the syringe pump to perform a discharging operation according to the position of the bubble:
outputting a bubble discharging prompt message;
controlling the syringe pump to perform the discharging operation according to discharging times or discharging volume indicated by an event that a user pressed a manual discharging key and is detected within a preset duration; and
controlling the syringe pump to perform the discharging operation according to the position of the bubble, so as to discharge the bubble from the syringe barrel of the syringe pump if the event is not detected within the duration.

10. A control device for discharging bubble in a syringe pump, comprising:
a detection module, configured to detect whether there is a bubble in a syringe barrel of the syringe pump through a bubble detection device;
a positioning module, configured to determine a position of the bubble if there is a bubble in the syringe barrel;
a control module, configured to control the syringe pump to perform a discharging operation according to the position of the bubble so as to discharge the bubble from the syringe barrel.

11. A syringe pump, comprising: a controller, a bubble detection device, a syringe, a push-pull device and a driving device; wherein
the controller is electrically coupled with the bubble detection device and the driving device;
the syringe comprises a syringe barrel, one end of the push-pull device abuts against an end of the syringe, and the other end of the push-pull device is connected to the driving device; and
the controller is configured to detect whether there is a bubble in the syringe barrel through the bubble detection device, determine a position of the bubble if there is a bubble in the syringe barrel, and control the push-pull device and the driving device to perform a discharging operation according to the position of the bubble so as to discharge the bubble from the syringe barrel.

12. The syringe pump according to claim 11, wherein the bubble detection device is a camera device, the syringe barrel comprises a main syringe barrel and an extension tube, one end of the extension tube is connected to the main syringe barrel; and
the controller is further configured to acquire an image of the syringe barrel captured by the camera device, identify a bubble in the image, and determine that there is a bubble in the syringe barrel if the bubble is identified in the image.

13. The method according to claim 12, wherein the bubble detection device comprises a plurality of camera devices respectively configured to shoot different parts of the syringe barrel; and
the controller is further configured to determine the target camera device that captured the image in which the bubble is identified if there is a bubble in the syringe barrel, and determine a target part of the syringe barrel corresponding to the target camera device as the position of the bubble according to a correspondence between the camera devices and different parts of the syringe barrel.

14. The method according to claim 13, wherein the syringe pump further comprises: a moving device;
the controller is further configured to acquire a size of the syringe barrel, and divide the syringe barrel into a plurality of parts according to a preset division rule;
the controller is further configured to determine target positions and target shooting parameters of the camera devices according to the size of the syringe barrel and positions of the divided parts;
the controller is further configured to determine and record the correspondence between the camera devices and the different parts of the syringe barrel according to the target positions; and
the controller is further configured to control the moving device to drive the camera devices to move to the target positions, and adjust shooting parameters of the camera devices to the target shooting parameters.

15. The syringe pump according to claim 11, further comprising a display and a manual discharging key, and the display and the manual discharging key are electrically connected to the controller;
the controller is further configured to output a bubble discharging prompt message to the display when it is detected that there is a bubble in the syringe barrel; and
the controller is further configured to control the push-pull device and the driving device to perform a discharging operation if an event that a user pressed the manual discharging key is detected within a preset duration.

16. An electronic device, comprising:
a memory and processor;
the memory stores executable program codes; and
the processor coupled to the memory is configured to invoke the executable program codes stored in the memory to execute the control method for discharging bubble in a syringe pump according to any one of claims 1-9.

17. A computer-readable storage medium, in which a computer program is stored, wherein when the computer program is executed by a processor, the control method for discharging bubble in a syringe pump according to any one of claims 1-9 is implemented.
